(19) Europäisches Patentamt
European Patent Office
Office européen des brevets

(11) **EP 2 716 623 A1**

(12) **EUROPÄISCHE PATENTANMELDUNG**


(43) Veröffentlichungstag:
**09.04.2014 Patentblatt 2014/15**

(51) Int Cl.:
***C07C 51/36*** (2006.01) ***C07C 61/08*** (2006.01)
***C07C 67/303*** (2006.01) ***C07C 69/75*** (2006.01)

(21) Anmeldenummer: **12187399.6**

(22) Anmeldetag: **05.10.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**



(71) Anmelder: **BASF SE**
**67056 Ludwigshafen (DE)**

(72) Erfinder:
- **Bock, Martin**
  **89231 Neu-Ulm (DE)**
- **Breitscheidel, Boris**
  **67165 Waldsee (DE)**

(74) Vertreter: **Steinbusch, Daniel**
**Isenbruck Bösl Hörschler LLP**
**EASTSITE ONE**
**Seckenheimer Landstrasse 4**
**68163 Mannheim (DE)**


(54) **Verfahren zur Herstellung von Cyclohexanpolycarbonsäure-Derivaten mit geringem Nebenproduktanteil**


(57) Die vorliegende Erfindung betrifft ein Verfahren zur Herstellung von wenigstens einer Cyclohexanpolycarbonsäure oder einem Derivat davon durch Inkontaktbringen wenigstens einer entsprechenden Benzolpolycarbonsäure oder eines Derivates davon mit einem Wasserstoff enthaltenden Gas in Gegenwart wenigstens eines Hydrierkatalysators, dadurch gekennzeichnet, dass das Inkontaktbringen bei einer Leerrohrgeschwindigkeit von höchstens 50 m/h erfolgt.


EP 2 716 623 A1

**Beschreibung**

Die vorliegende Erfindung betrifft ein Verfahren zur Herstellung von wenigstens einer Cyclohexanpolycarbonsäure oder einem Derivat davon durch Inkontaktbringen wenigstens einer entsprechenden Benzolpolycarbonsäure oder eines Derivates davon mit einem Wasserstoff enthaltenden Gas in Gegenwart wenigstens eines Hydrierkatalysators, dadurch gekennzeichnet, dass das Inkontaktbringen bei einer Leerrohrgeschwindigkeit von höchstens 50 m/h erfolgt.

Ferner betrifft die vorliegende Erfindung auch ausgewählte Vertreter der erhaltenen Cyclohexanpolycarbonsäuren oder einem Derivat davon, sowie die Verwendung der erhaltenen Cyclohexanpolycarbonsäuren oder einem Derivat davon als Weichmacher in Kunststoffen.

In der US 5,286,898 und der US 5,319,129 wird Dimethylterephthalat an geträgerten Pd-Katalysatoren, die mit Ni, Pt und/oder Ru versetzt sind, bei Temperaturen > 140 °C und einem Druck zwischen 50 und 170 bar zum entsprechenden Hexahydrodimethylterephthalat hydriert. In der DE-A 28 23 165 werden aromatische Carbonsäureester an geträgerten Ni-, Ru-, Rh-, und/oder Pd-Katalysatoren zu den entsprechenden cycloaliphatischen Carbonsäureestern bei 70 bis 250 °C und 30 bis 200 bar hydriert. In der US 3,027,398 wird die Hydrierung von Dimethylterephthalat an geträgerten Ru-Katalysatoren bei 110 bis 140 °C und 35 bis 105 bar beschrieben.

Die EP-A 0 603 825 betrifft ein Verfahren zur Herstellung von 1,4-Cyclohexandicarbonsäure durch Hydrierung von Terephthalsäure unter Verwendung eines geträgerten PalladiumKatalysators, wobei als Träger Aluminiumoxid, Siliziumdioxid oder Aktivkohle verwendet wird. Das dort beschriebene Verfahren ist insbesondere dadurch charakterisiert, dass die in einer ersten Stufe erhaltene 1,4-Cyclohexandicarbonsäure enthaltende Lösung mit Dampf in Kontakt gebracht wird und dadurch in dieser Lösung enthaltene Verunreinigungen extrahiert werden. Dieses Verfahren ist jedoch nur auf Säuren anwendbar, da bei der Anwendung auf Derivate, wie z. B. Ester, Anhydride, usw. die Gefahr von Hydrolyse besteht. In der EP 1 042 273 ist ein Verfahren zur Hydrierung von Polycarbonsäure-Derivaten unter Verwendung eines Makroporen aufweisenden Katalysators beschrieben. Das Verfahren zeichnet sich durch eine hohe Raum-ZeitAusbeute und eine hohe Selektivität aus.

Einige Cyclohexanpolycarbonsäure-Derivate sowie deren Verwendung als Weichmacher sind ebenfalls aus dem Stand der Technik bekannt. So sind Cyclohexandicarbonsäuredimethyl- oder diethylester (DE-A 28 23 165), Cyclohexan-1,2-dicarbonsäuredi(2-ethylhexyl)ester (DE-A 12 63 296) und Cyclohexan-1,2-dicarbonsäurediisononylester (EP 1 042 273) und deren Verwendung als Weichmacher in Kunstoffen beschrieben.

Der Nachteil der oben beschriebenen Herstellverfahren bzw. der mit Hilfe dieser Verfahren hergestellten Cyclohexanpolycarbonsäure-Derivate besteht in einem hohen Anteil von Nebenprodukten im Endprodukt, insbesondere einem hohen Anteil an Hexahydrophthalid und Isononylalkohol, die ggf. eine aufwendige Nachreinigung erfordert. Bedingt durch diesen hohen Anteil an Nebenprodukten weisen die durch Verfahren gemäß Stand der Technik hergestellten Cyclohexanpolycarbonsäure-Derivate bei der Verwendung als Weichmacher nachteilige anwendungstechnische Eigenschaften auf, wie eine hohe Flüchtigkeit und eine schlechte Verträglichkeit mit Kunststoffen, beispielweise PVC. Dadurch sind die aus dem Stand der Technik bekannten Cyclohexanpolycarbonsäure-Derivate weniger gut für sensible Anwendungen in Kontakt mit Menschen geeignet, z. B. für Kinderspielzeug, Lebensmittelverpackungen oder medizinische Artikel.

Der vorliegenden Erfindung lag die Aufgabe zugrunde, ein Verfahren zur Hydrierung von Benzolpolycarbonsäuren oder Derivaten davon zur Verfügung zu stellen, mit dem die entsprechenden Cyclohexanpolycarbonsäuren oder Derivate davon in hoher Reinheit, d. h. mit einem geringen Anteil an Nebenkomponenten, insbesondere mit einem niedrigen Anteil an Hexahydrophthalid und Isononylalkohol, erhalten werden können.

Eine weitere Aufgabe der vorliegenden Erfindung lag in der Bereitstellung ausgewählter Cyclohexanpolycarbonsäure-Derivate, die durch die erfindungsgemäße Hydrierung der entsprechenden Benzolpolycarbonsäure-Derivate erhältlich sind, und die sich besonders zur Verwendung als Weichmacher in Kunststoffen eignen, insbesondere für sensible Anwendungen im Kontakt mit Menschen.

Es wurde gefunden, dass man Cyclohexanpolycarbonsäuren oder Derivate davon mit einem verringerten Anteil an Nebenprodukten, insbesondere Hexahydrophthalid und Isononanol, erhält, wenn die Hydrierung der entsprechenden Benzolpolycarbonsäuren oder deren Derivate, d. h. das Inkontaktbringen dieser Verbindungen mit einem Wasserstoff enthaltenden Gas, bei einer Leerrohrgeschwindigkeit von höchstens 50 m/h erfolgt.

Demgemäß betrifft die vorliegende Erfindung ein Verfahren zur Herstellung von wenigstens einer Cyclohexanpolycarbonsäure oder eines Derivates davon durch Inkontaktbringen wenigstens einer entsprechenden Benzolpolycarbonsäure oder eines Derivates davon mit einem Wasserstoff enthaltenden Gas in Gegenwart wenigstens eines Hydrierkatalysators, wobei das Inkontaktbringen bei einer Leerrohrgeschwindigkeit von höchstens 50 m/h erfolgt.

Das erfindungsgemäße Inkontaktbringen der wenigstens einen Cyclohexanpolycarbonsäure oder eines Derivates davon mit einem Wasserstoff enthaltenden Gas resultiert in einer Hydrierung dieser Verbindungen, um die gewünschte wenigstens eine Cyclohexanpolycarbonsäure oder ein Derivat davon zu erhalten. Erfindungsgemäß bevorzugt wird nur das aromatische System hydriert, d. h. reduziert, um das entsprechende gesättigte cycloaliphatische System zu erhalten, d. h. ggf. weitere in dem wenigstens einen Substrat vorliegende reduzierbare Gruppen werden erfindungsgemäß bevorzugt nicht reduziert.

Für das erfindungsgemäße Verfahren geeignete Katalysatoren enthalten mindestens ein Aktivmetall, welches wahlweise als Vollkontakt oder fixiert auf einem Träger eingesetzt werden kann.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium oder Nickel als Aktivmetall verwendet werden. Unter den ebenfalls verwendbaren Metallen der I., II. oder VII. Nebengruppe des Periodensystems, die ebenfalls allesamt prinzipiell verwendbar sind, werden vorzugsweise Kupfer, Zink und/oder Rhenium eingesetzt.

Entsprechende Katalysatoren sind zum Beispiel in Ullmann's Encyclopedia of Industrial Chemistry Vol. 18, Kapitel Hydrogenation and Dehydrogenation, Seiten 483-541, beschrieben.

Geeignete Aktivmetalle können auf einem inerten Träger aufgebracht sein, geeignete Trägermaterialien sind z. B. Aktivkohlen, Metalloxide und Zeolithe. Bevorzugt sind Katalysatoren die als Trägermaterial Aluminiumoxid, Siliziumdioxid, Kohle oder Mischoxide die Aluminiumoxid und/oder Siliziumdioxid enthalten. Geeignete Materialien sind z. B. in Ullmann's Encyclopedia of Industrial Chemistry Vol . 17, Kapitel Heterogeneous Catalysis and Solid Catalysts, 2. Development and Types of Solid Catalysts, Seiten 483-541, beschrieben.

Der Gehalt des Aktivmetalls beträgt im Fall von Platinmetallen (Ruthenium, Rhodium, Palladium, Platin) im allgemeinen 0,01 bis 30 Gew.-%, vorzugsweise 0,01 bis 5 Gew.-% und insbesondere 0,1 bis 5 Gew.-%, jeweils bezogen auf das Gesamtgewicht des verwendeten Katalysators, wobei die bei den im folgenden beschriebenen, vorzugsweise eingesetzten Katalysatoren 1 bis 3 vorzugsweise verwendeten Gehalte nochmals bei der Diskussion dieser Katalysatoren einzeln angegeben sind. Im Falle von Nickel oder Cobalt als Aktivmetall beträgt der Aktivmetallgehalt im Allgemeinen zwischen 5 und 100 Gew-%, bezogen auf das Gesamtgewicht des Katalysators.

Der erfindungsgemäß verwendete Begriff *Benzolpolycarbonsäure oder ein Derivat davon* umfasst alle Benzolpolycarbonsäuren an sich, wie z. B. Phthalsäure, Isophthalsäure, Terephthalsäure, Trimellitsäure, Trimesinsäure, Hemimellitsäure und Pyrromellitsäure, als auch Derivate davon, wobei insbesondere Mono-, Di- und ggf. Tri- und Tetraester, insbesondere Alkylester, und Anhydride zu nennen sind. Die vorzugsweise eingesetzten Verbindungen werden untenstehend im Abschnitt *Die Verfahrensführung* detailliert erläutert.

Im Folgenden sollen nunmehr die erfindungsgemäß vorzugsweise verwendeten Katalysatoren detailliert beschrieben werden. Dabei erfolgt die Beschreibung beispielhaft unter Bezugnahme auf die Verwendung von Ruthenium als Aktivmetall. Die untenstehenden Angaben sind auch auf die anderen verwendbaren Aktivmetalle, wie hierin definiert, übertragbar.

Katalysator 1

Die erfindungsgemäß verwendeten Katalysatoren 1 können technisch hergestellt werden durch Auftragen mindestens eines Metalls der VIII. Nebengruppe des Periodensystems und gegebenenfalls mindestens eines Metalls der I. oder VII. Nebengruppe des Periodensystems auf einen geeigneten Träger.

Die Auftragung kann durch Tränken des Trägers in wässrigen Metallsalzlösungen, wie z. B. wässrigen Rutheniumsalzlösungen, durch Aufsprühen entsprechender Metallsalzlösungen auf den Träger oder durch andere geeignete Verfahren erreicht werden. Als Metallsalze der I., VII. oder VIII. Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der VIII. Nebengruppe des Periodensystems noch weitere Metalle als Aktivmetall auf dem Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend, vorzugsweise bei Temperaturen von 100 bis 150 °C, getrocknet und wahlweise bei Temperaturen von 200 bis 600 °C, vorzugsweise von 350 bis 450 °C calziniert. Bei getrennter Auftränkung wird der Katalysator nach jedem Tränkschritt getrocknet und wahlweise calziniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen von 30 bis 600 °C, vorzugsweise von 150 bis 450 °C aktiviert. Vorzugsweise besteht der Gasstrom aus 50 bis 100 Vol.-% $H_2$ und 0 bis 50 Vol.-% $N_2$.

Die Metallsalzlösung oder -lösungen werden in einer solchen Menge auf den oder die Träger aufgebracht, dass der Gesamtgehalt an Aktivmetall, jeweils bezogen auf das Gesamtgewicht des Katalysators, 0,01 bis 30 Gew.-%, vorzugsweise 0,01 bis 5 Gew.-%, weiter bevorzugt 0,01 bis 1 Gew.-%, und insbesondere 0,05 bis 1 Gew.-% beträgt.

Die Metalloberfläche auf dem Katalysator 1 beträgt dabei insgesamt vorzugsweise 0,01 bis 10 $m^2/g$, weiter bevorzugt 0,05 bis 5 $m^2/g$ und insbesondere 0,05 bis 3 $m^2/g$ des Katalysators. Die Metalloberfläche wird mittels der von J. Lemaitre et al. in Characterization of Heterogeneous Catalysts, Hrsg. Francis Delanney, Marcel Dekker, New York

1984, S. 310 - 324, beschriebenen Chemisorptionsverfahren bestimmt.

Im erfindungsgemäß verwendeten Katalysator 1 beträgt das Verhältnis der Oberflächen des/der Aktivmetalls/-metalle und des Katalysatorträgers vorzugsweise weniger als 0,05, wobei der untere Grenzwert bei 0,0005 liegt.

Die zur Herstellung der erfindungsgemäß verwendeten Katalysatoren verwendbaren Trägermaterialien sind solche, die makroporös sind und einen mittleren Porendurchmesser von mindestens 50 nm, vorzugsweise mindestens 100 nm, insbesondere mindestens 500 nm aufweisen und deren Oberfläche nach BET bei höchstens 30 $m^2/g$, vorzugsweise höchstens 15 $m^2/g$, weiter bevorzugt höchstens 10 $m^2/g$, insbesondere höchstens 5 $m^2/g$ und weiter bevorzugt höchstens 3 $m^2/g$ liegt. Der mittlere Porendurchmesser des Trägers beträgt vorzugsweise 100 nm bis 200 $\mu$m, weiter bevorzugt 500 nm bis 50 $\mu$m. Die Oberfläche des Trägers beträgt vorzugsweise 0,2 bis 15 $m^2/g$, weiter bevorzugt 0,5 bis 10 $m^2/g$, insbesondere 0,5 bis 5 $m^2/g$ und weiter bevorzugt 0,5 bis 3 $m^2/g$.

Die Oberfläche des Trägers wird bestimmt nach dem BET-Verfahren durch $N_2$-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 $\mu$m bei der bimodalen Verteilung eine spezielle Ausführungsform der Erfindung darstellt.

Weiter bevorzugt ist ein Träger mit einer Oberfläche von 1,70 bis 180 $m^2/g$, beispielsweise 1,75 $m^2/g$, der diese bimodale Verteilung des Porendurchmessers aufweist. Das Porenvolumen dieses bevorzugten Trägers beträgt vorzugsweise etwa 0,50 bis 0,60 ml/g, beispielsweise 0,53 ml/g.

Als makroporöses Trägermaterial verwendbar sind beispielsweise Makroporen aufweisende Aktivkohle, Siliziumcarbid, Aluminiumoxid, Siliziumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Weitere Details bezüglich Katalysator 1 bzw. zu seiner Herstellung sind der DE-A 25 196 24 484 zu entnehmen, deren diesbezüglicher Inhalt durch Bezugnahme vollständig in die vorliegende Anmeldung einbezogen wird.

Katalysator 2

Die erfindungsgemäß verwendeten Katalysatoren 2 enthalten ein oder mehrere Metalle der VIII. Nebengruppe des Periodensystems als Aktivkomponente(n) auf einem Träger, wie hierin definiert. Bevorzugt werden Ruthenium, Palladium und/oder Rhodium als Aktivkomponente(n) verwendet.

Die erfindungsgemäß verwendeten Katalysatoren 2 können technisch hergestellt werden durch Auftragen mindestens eines Aktivmetalls der VIII. Nebengruppe des Periodensystems, vorzugsweise Ruthenium oder Palladium und gegebenenfalls mindestens eines Metalls der I. oder VII. Nebengruppe des Periodensystems auf einen geeigneten Träger. Die Auftragung kann durch Tränken des Trägers in wässrigen Metallsalzlösungen, wie z.B. Ruthenium- oder Palladiumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen von 100 bis 150 °C bevorzugt sind. Wahlweise können diese Träger bei Temperaturen von 200 bis 600 °C, vorzugsweise von 350 bis 450 °C calziniert werden. Anschließend werden die beschichteten Träger durch Behandlung in einem Gasstrom, der freien Wasserstoff enthält, bei Temperaturen von 30 bis 600 °C, vorzugsweise von 100 bis 450 °C und insbesondere von 100 bis 300 °C aktiviert. Der Gasstrom besteht vorzugsweise aus 50 bis 100 Vol.-% $H_2$ und 0 bis 50 Vol.-% $N_2$.

Werden auf die Träger mehrere Aktivmetalle aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calziniert werden. Dabei kann die Reihenfolge, in der die Metallsalzlösung aufgetragen oder aufgetränkt wird, beliebig gewählt werden.

Die Metallsalzlösung wird in einer solchen Menge auf den/die Träger aufgebracht, dass der Gehalt an Aktivmetall 0,01 bis 30 Gew.-%, vorzugsweise 0,01 bis 10 Gew.-%, weiter bevorzugt 0,01 bis 5 Gew.-%, und insbesondere 0,3 bis 1 Gew.-%, bezogen auf das Gesamtgewicht des Katalysators, beträgt.

Die Metalloberfläche auf dem Katalysator beträgt insgesamt vorzugsweise 0,01 bis 10 $m^2/g$, besonders bevorzugt 0,05 bis 5 $m^2/g$ und weiter bevorzugt 0,05 bis 3 $m^2/g$ des Katalysators. Die Metalloberfläche wurde durch das Chemisorptionsverfahren gemessen, wie es in J. Lemaitre et al., Characterization of Heterogeneous Catalysts, Hrsg. Francis Delanney, Marcel Dekker, New York (1984), S. 310 - 324, beschrieben ist.

Im erfindungsgemäß verwendeten Katalysator 2 beträgt das Verhältnis der Oberflächen des mindestens einen

Aktivmetalls und des Katalysatorträgers weniger als 0,3, vorzugsweise weniger als 0,1 und insbesondere 0,05 oder weniger, wobei der untere Grenzwert bei 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß bevorzugt verwendbaren Träger eine Porenverteilung auf, der gemäß 5 bis 50%, vorzugsweise 10 bis 45%, weiter bevorzugt 10 bis 30% und insbesondere 15 bis 25% des Porenvolumens von Makroporen mit Porendurchmessern im Bereich von 50 nm bis 10.000 nm und 50 bis 95%, vorzugsweise 55 bis 90%, weiter bevorzugt 70 bis 90% und insbesondere 75 bis 85% des Porenvolumens von Mesoporen mit einem Porendurchmesser von 2 bis 50 nm gebildet werden, wobei sich jeweils die Summe der Anteile der Porenvolumina zu 100% addiert.

Das Gesamtporenvolumen der erfindungsgemäß verwendeten Träger beträgt 0,05 bis 1,5 $cm^3$/g, vorzugsweise 0,1 bis 1,2 $cm^3$/g und insbesondere 0,3 bis 1,0 $cm^3$/g. Der mittlere Porendurchmesser der erfindungsgemäß verwendeten Träger beträgt 5 bis 20 nm, vorzugsweise 8 bis 15 nm und insbesondere 9 bis 12 nm.

Vorzugsweise beträgt die Oberfläche des Trägers 50 bis 500 $m^2$/g, weiter bevorzugt 200 bis 350 $m^2$/g und insbesondere 250 bis 300 $m^2$/g des Trägers.

Die Oberfläche des Trägers wird nach dem BET-Verfahren durch $N_2$-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d. h. die die oben definierte Porengrößenverteilung aufweisen, eingesetzt werden können, werden vorzugsweise Makroporen aufweisende Aktivkohle, Siliziumcarbid, Aluminiumoxid, Siliziumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich Katalysators 2 bzw. zu seiner Herstellung sind der DE-A 196 24 485 zu entnehmen, deren diesbezüglicher Inhalt durch Bezugnahme vollständig in die vorliegende Anmeldung einbezogen wird.

Katalysator 3

Die erfindungsgemäß verwendeten Katalysatoren 3 können technisch hergestellt werden durch Auftragen eines Aktivmetalls der VIII. Nebengruppe des Periodensystems und gegebenenfalls mindestens eines Metalls der I. oder VII. Nebengruppe des Periodensystems auf einen geeigneten Träger. Die Auftragung kann durch Tränken des Trägers in wässrigen Metallsalzlösungen, wie z. B. Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I., VII. oder VIII. Nebengruppe eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, bevorzugt sind dabei die Nitrate und Nitrosylnitrate.

Bei Katalysatoren, die mehrere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen von 100 bis 150 °C, und wahlweise bei Temperaturen von 200 bis 600 °C calziniert.

Darauffolgend werden die beschichteten Träger aktiviert durch Behandlung der beschichteten Träger in einem Gasstrom, der freien Wasserstoff enthält, bei Temperaturen von 30 bis 600 °C, vorzugsweise von 150 bis 450 °C. Der Gasstrom besteht vorzugsweise aus 50 bis 100 Vol.-% $H_2$ und 0 bis 50 Vol.-% $N_2$.

Werden auf die Träger neben dem Aktivmetall der VIII. Nebengruppe des Periodensystems Metalle der I. oder VII. Nebengruppe aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calziniert werden. Dabei kann die Reihenfolge, in der die Metallsalzlösungen aufgetragen oder aufgetränkt werden, beliebig gewählt werden.

Die Metallsalzlösung wird in einer solchen Menge auf den oder die Träger aufgebracht, dass 0,01 bis 30 Gew.-%, bezogen auf das Gesamtgewicht des Katalysators, an Aktivmetall auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator 3 beträgt insgesamt vorzugsweise 0,01 bis 10 $m^2$/g, besonders bevorzugt 0,05 bis 5 $m^2$/g, insbesondere 0,05 bis 3 $m^2$ pro g des Katalysators.

Die zur Herstellung der erfindungsgemäß verwendeten Katalysatoren 3 verwendbaren Trägermaterialien sind vorzugsweise solche, die makroporös sind und einen mittleren Porendurchmesser von mindestens 0,1 µm, vorzugsweise mindestens 0,5 µm, und eine Oberfläche von höchstens 15 $m^2$/g aufweisen, vorzugsweise höchstens 10 $m^2$/g, besonders bevorzugt höchstens 5 $m^2$/g, insbesondere höchstens 3 $m^2$/g.

Bevorzugt liegt der mittlere Porendurchmesser des Trägers in einem Bereich von 0,1 bis 200 µm, insbesondere von 0,5 bis 50 µm. Bevorzugt beträgt die Oberfläche des Trägers 0,2 bis 15 $m^2$/g, besonders bevorzugt 0,5 bis 10 $m^2$/g,

insbesondere 0,5 bis 5 $m^2/g$, speziell 0,5 bis 3 $m^2/g$ des Trägers.

Die Oberfläche des Trägers wird bestimmt nach dem BET-Verfahren durch $N_2$-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesser-verteilung mit Maxima bei etwa 0,6 $\mu$m und etwa 20 $\mu$m bei der bimodalen Verteilung eine spezielle Ausführungsform der Erfindung darstellt.

Besonders bevorzugt ist ein Träger mit einer Oberfläche von etwa 1,75 $m^2/g$, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliziumcarbid, Aluminiumoxid, Siliziumdioxid, Titandioxid, Zirkoniumdioxid, Magnesiumoxid, Zinkoxid oder deren Gemische. Bevorzugt sind Aluminiumoxid und Zirkoniumdioxid.

Weitere Details bezüglich Katalysator 3 bzw. zu seiner Herstellung sind der DE-A 196 04 791.9 zu entnehmen, deren diesbezüglicher Inhalt durch Bezugnahme vollständig in die vorliegende Anmeldung einbezogen wird.

Katalysator 4:

Erfindungsgemäß besonders geeignet sind Schalenkatalysatoren (Katalysatoren 4) mit einem Aktivmetall auf einem Träger. Entsprechende Schalenkatalysatoren sind in der WO2011/08299 genannt. Der Inhalt der WO2011/08299 wird durch Bezugnahme vollständig in die vorliegende Anmeldung einbezogen.

Besonders bevorzugt ist ein entsprechender Schalenkatalysator, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2/g$ beträgt, und mindestens 90% der vorhandenen Poren einen Durchmesser von 6 bis 12 nm aufweisen.

Die vorliegende Erfindung betrifft daher bevorzugt das erfindungsgemäße Verfahren, wobei als Katalysator ein Schalenkatalysator, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2/g$ beträgt, und mindestens 90% der vorhandenen Poren einen Durchmesser von 6 bis 12 nm aufweisen, eingesetzt wird.

Der bevorzugt eingesetzte Schalenkatalysator enthält ein Aktivmetall ausgewählt aus der Gruppe bestehend aus Ruthenium, Rhodium, Palladium, Platin und Mischungen davon, bevorzugt Ruthenium, ganz besonders bevorzugt Ruthenium als einziges Aktivmetall.

In dem erfindungsgemäß bevorzugt eingesetzten Schalenkatalysator beträgt die Menge des Aktivmetalls im Allgemeinen weniger als 1 Gew.-%, bevorzugt 0,1 bis 0,5 Gew.-%, besonders bevorzugt 0,25 bis 0,35 Gew.-%, bezogen auf das Gesamtgewicht des Katalysators.

Schalenkatalysatoren sind dem Fachmann an sich bekannt. Im Rahmen der vorliegenden Erfindung bedeutet der Begriff "Schalenkatalysator", dass das vorhandene mindestens eine Aktivmetall, bevorzugt Ruthenium, zum überwiegenden Teil in einer äußeren Schale des Trägermaterials vorliegt.

In dem erfindungsgemäß bevorzugt eingesetzten Schalenkatalysatoren liegen bevorzugt 40 bis 70 Gew.-%, besonders bevorzugt 50 bis 60 Gew.-% des Aktivmetalls, bezogen auf die Gesamtmenge des Aktivmetalls, in der Schale des Katalysators bis zu einer Eindringtiefe von 200 $\mu$m vor. In einer besonders bevorzugten Ausführungsform liegen 60 bis 90 Gew.- %, ganz besonders bevorzugt 70 bis 80 Gew.-% des Aktivmetalls, bezogen auf die Gesamtmenge des Aktivmetalls, in der Schale des Katalysators bis zu einer Eindringtiefe von 500 $\mu$m vor. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Weitere Informationen bezüglich den vorstehend genannten Messverfahren und Techniken sind zum Beispiel in Spectroscopy in Catalysis von J.W. Niemantsverdriet, VCH, 1995 oder Handbook of Microscopy von S. Amelinckx et al. offenbart. Zur Ermittlung der Eindringtiefe der Aktivmetallteilchen werden mehrere Katalysatorteilchen (z. B. 3, 4 oder 6) angeschliffen. Mittels Linescans werden dann die Profile der Aktivmetall/Si Konzentrationsverhältnisse erfasst. Auf jeder Messlinie werden mehrere, zum Beispiel 15 bis 20, Messpunkte in gleichen Abständen gemessen, die Messfleckgröße beträgt circa 10 $\mu$m * 10 $\mu$m. Nach Integration der Aktivmetallmenge über die Tiefe kann die Häufigkeit des Aktivmetalls in einer Zone bestimmt werden.

Ganz besonders bevorzugt beträgt die Menge des Aktivmetalls, bezogen auf das Konzentrationsverhältnis von Aktivmetall zu Si, an der Oberfläche des Schalenkatalysators, 2 bis 25%, bevorzugt 4 bis 10%, besonders bevorzugt 4 bis 6%, ermittelt mittels SEM EPMA - EDXS. Die Oberflächenanalyse erfolgt mittels Bereichsanalysen von Bereichen von 800 $\mu$m * 2000 $\mu$m und mit einer Informationstiefe von circa 2 $\mu$m. Die Elementzusammensetzung wird in Gew.-% (normiert auf 100 Gew.-%) bestimmt. Das mittlere Konzentrationsverhältnis (Aktivmetall/Si) wird über 10 Messbereiche gemittelt.

Unter der Oberfläche des Schalenkatalysators ist im Sinne der vorliegenden Erfindung die äußere Schale des Katalysators bis zu einer Eindringtiefe von circa 2 µm zu verstehen. Diese Eindringtiefe entspricht der Informationstiefe bei der vorstehend erwähnten Oberflächenanalyse.

Ganz besonders bevorzugt ist ein Schalenkatalysator, worin die Menge des Aktivmetalls, bezogen auf das Gewichtsverhältnis von Aktivmetall zu Si (Gew./Gew. in%), an der Oberfläche des Schalenkatalysators 4 bis 6% beträgt, in einer Eindringtiefe von 50 µm 1,5 bis 3% und im Bereich von 50 bis 150 µm Eindringtiefe 0,5 bis 2%, ermittelt mittels SEM EPMA (EDXS), beträgt. Die genannten Werte stellen gemittelte Werte dar.

Des Weiteren nimmt die Größe der Aktivmetallteilchen bevorzugt mit zunehmender Eindringtiefe ab, ermittelt mittels (FEG)-TEM-Analyse.

Das Aktivmetall liegt in dem erfindungsgemäßen Schalenkatalysator bevorzugt teilweise oder vollständig kristallin vor. In bevorzugten Fällen kann in der Schale des erfindungsgemäßen Schalenkatalysators mittels SAD (Selected Area Diffraction) feinstkristallines Aktivmetall nachgewiesen werden.

Der erfindungsgemäß bevorzugte Schalenkatalysator kann zusätzlich Erdalkalimetallionen ($M^{2+}$), also M = Be, Mg, Ca, Sr und/oder Ba, insbesondere Mg und/oder Ca, ganz besonders Mg enthalten. Der Gehalt an Erdalkalimetallion/en ($M^{2+}$) im Katalysator beträgt bevorzugt 0,01 bis 1 Gew.-%, insbesondere 0,05 bis 0,5 Gew.-%, ganz besonders 0,1 bis 0,25 Gew.-%, jeweils bezogen auf das Gewicht des Siliziumdioxid-Trägermaterials.

Ein wesentlicher Bestandteil der erfindungsgemäß bevorzugten Katalysatoren ist das Trägermaterial enthaltend Siliziumdioxid, bevorzugt amorphes Siliziumdioxid. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden.

Als Trägermaterialien kommen grundsätzlich amorphe Siliziumdioxid-Typen in Betracht, die mindestens zu 90 Gew.-% aus Siliziumdioxid bestehen, wobei die verbleibenden 10 Gew.-%, vorzugsweise nicht mehr als 5 Gew.-%, des Trägermaterials auch ein anderes oxidisches Material sein können, z. B. MgO, CaO, $TiO_2$, $ZrO_2$ und/oder $Al_2O_3$.

In einer bevorzugten Ausführungsform der Erfindung ist das Trägermaterial halogenfrei, insbesondere chlorfrei, d. h. der Gehalt an Halogen im Trägermaterial beträgt im Allgemeinen weniger als 500 Gew.-ppm, z. B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält. Besonders bevorzugt liegt der Halogenidgehalt des Trägermaterials unterhalb der analytischen Nachweisgrenze. Bevorzugt sind Trägermaterialien enthaltend Siliziumdioxid, die eine spezifische Oberfläche im Bereich von 280 bis 500 $m^2/g$, besonders bevorzugt 280 bis 400 $m^2/g$, ganz besonders bevorzugt 300 bis 350 $m^2/g$, (BET-Oberfläche nach DIN 66131) aufweisen. Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf.

Das Porenvolumen des Trägermaterials beträgt erfindungsgemäß 0,6 bis 1,0 ml/g, bevorzugt 0,65 bis 0,9 ml/g, beispielsweise 0,7 bis 0,8 ml/g, bestimmt durch Hg-Porosimetrie (DIN 66133). In dem erfindungsgemäß bevorzugten Schalenkatalysator weisen mindestens 90% der vorhandenen Poren einen Porendurchmesser von 6 bis 12 nm, bevorzugt 7 bis 11 nm, besonders bevorzugt 8 bis 10 nm, auf. Der Porendurchmesser kann nach dem Fachmann bekannten Verfahren bestimmt werden, beispielsweise durch Hg-Porosimetrie oder $N_2$-Physisorption. In einer bevorzugten Ausführungsform weisen mindestens 95%, besonders bevorzugt mindestens 98%, der vorhandenen Poren einen Porendurchmesser von 6 bis 12 nm, bevorzugt 7 bis 11 nm, besonders bevorzugt 8 bis 10 nm, auf.

In dem erfindungsgemäß bevorzugten Schalenkatalysator liegen in einer bevorzugten Ausführungsform keine Poren vor, die kleiner als 5 nm sind. Des Weiteren liegen in dem erfindungsgemäß bevorzugten Schalenkatalysator keine Poren vor, die grösser als 25 nm, insbesondere grösser als 15 nm, sind. In diesem Zusammenhang bedeutet "keine Poren", dass mit üblichen Messverfahren, beispielsweise Hg-Porosimetrie oder $N_2$-Physisorption keine Poren mit diesen Durchmessern gefunden werden. In dem erfindungsgemäß bevorzugten Schalenkatalysator liegen im Rahmen der Messgenauigkeit der verwendeten Analytik keine Makroporen, sondern ausschließlich Mesoporen vor.

Bei dem erfindungsgemäß bevorzugten Schalenkatalysators werden besonders bevorzugt Formkörper aus dem Trägermaterial, die z. B. durch Extrudieren, Strangpressen oder Tablettieren erhältlich sind und die z. B. die Form von Kugeln, Tabletten, Zylindern, Strängen, Ringen bzw. Hohlzylindern, Sternen und dergleichen, besonders bevorzugt Kugeln, aufweisen können, eingesetzt. Die Abmessungen dieser Formkörper bewegen sich üblicherweise im Bereich von 0,5 mm bis 25 mm. Bevorzugt werden Katalysatorkugeln mit Kugeldurchmessern von 1,0 bis 6,0 mm, besonders bevorzugt 2,5 bis 5,5 mm, eingesetzt. In dem erfindungsgemäß bevorzugten Schalenkatalysator beträgt Dispersität des Aktivmetalls bevorzugt 30 bis 60%, besonders bevorzugt 30 bis 50%. Verfahren zur Messung der Dispersität des Aktivmetalls sind dem Fachmann an sich bekannt, beispielsweise durch Puls-Chemisorption, wobei die Bestimmung der Edelmetalldispersion (spezifische Metalloberfläche, Kristallitgrösse) mit CO Pulsmethode durchgeführt wird (DIN 66136(1-3)).

In dem erfindungsgemäß bevorzugten Schalenkatalysator beträgt die Oberfläche des Aktivmetalls bevorzugt

0,2 bis 0,8 $m^2/g$, besonders bevorzugt 0,3 bis 0,7 $m^2/g$. Verfahren zur Messung der Oberfläche des Aktivmetalls sind dem Fachmann an sich bekannt.

Die Herstellung der erfindungsgemäß bevorzugten Schalenkatalysatoren erfolgt beispielsweise dadurch, dass man zunächst das Trägermaterial mit einer Lösung enthaltend eine Vorläuferverbindung des Aktivmetalls ein- oder mehrfach tränkt, den erhaltenen Feststoff trocknet und anschließend reduziert. Die einzelnen Verfahrensschritte sind dem Fachmann an sich bekannt und in der WO 2011/082991 beschrieben.

Katalysator 5:

Erfindungsgemäß geeignet sind ebenfalls Katalysatoren 5 mit einem Aktivmetall auf einem mesoporösen Träger gemäß WO 2004/046078. Katalysator 5 enthält ein oder mehrere katalytisch aktiven Metall(e), wie Platin, Palladium, Ruthenium oder Mischungen davon, abgeschieden auf einem Katalysatorträgermaterial, umfassend ein oder mehrere geordnete mesoporöse Materialien. Der Inhalt der WO 2004/046078 wird durch Bezugnahme vollständig in die vorliegende Anmeldung einbezogen.

Katalysator 6:

Erfindungsgemäß geeignet sind ebenfalls Katalysatoren 6 mit einem Aktivmetall auf einem mesoporösen Träger gemäß US 7,893,295. Der darin genannte Katalysator enthält ein oder mehrere katalytisch aktive Metall(e), wie Platin, Palladium Ruthenium oder Mischungen davon, abgeschieden auf einem Katalysatorträgermaterial, umfassend ein oder mehrere geordnete mesoporöse Materialien. Der Inhalt der US 7,893,295 wird durch Bezugnahme vollständig in die vorliegende Anmeldung einbezogen.

Katalysator 7:

Erfindungsgemäß geeignet sind ebenfalls mesoporöse Metall/Träger-Katalysatoren 7 gemäß DE 10225565. Dieser Katalysator enthält mindestens ein Metall der Gruppe 8 auf oder in einem Trägermaterial mit einem mittleren Porendurchmesser von 25 bis 50 nm und einer spezifischen Oberfläche von mehr als 30 $m^2/g$. Der Inhalt der DE 10225565 wird durch Bezugnahme vollständig in die vorliegende Anmeldung einbezogen.

Katalysator 8:

Erfindungsgemäß geeignet sind ebenfalls Katalysatoren 8 auf Basis Nickel/Zink gemäß DE 10146847. Der Inhalt der DE 10146847 wird durch Bezugnahme vollständig in die vorliegende Anmeldung einbezogen.

Katalysator 9:

Erfindungsgemäß geeignet sind ebenfalls mikroporöse Metall-Katalysatoren 9 auf Titandioxid-Trägern gemäß WO04/009526. Der Inhalt der WO04/009526 wird durch Bezugnahme vollständig in die vorliegende Anmeldung einbezogen.

Die Verfahrensführung:

Für das erfindungsgemäße Verfahren ist es erfindungswesentlich, dass das Inkontaktbringen der wenigstens einen Benzolpolycarbonsäure oder eines Derivates davon mit dem Wasserstoff enthaltenden Gas, d. h. die Hydrierung der wenigstens einen Benzolpolycarbonsäure oder eines Derivates davon, bei einer Leerohrgeschwindigkeit von höchstens 50 m/h im ersten Reaktor erfolgt, besonders bevorzugt wird das erfindungsgemäße Verfahren bei einer Leerohrgeschwindigkeit von höchstens 40 m/h, durchgeführt. Das erfindungsgemäße Verfahren wird im Allgemeinen bei einer Leerrohrgeschwindigkeit von mindestens 20 m/h durchgeführt.

Die Leerrohrgeschwindigkeit ist erfindungsgemäß wie folgt definiert:

$$\text{Leerrohrgeschwindigkeit} = V(\text{Zulauf})/(A(1.\ \text{Reaktor})*t),$$

wobei V(Zulauf 1. Reaktor) die Summe des Volumens des Eduktes, d. h. der wenigstens einen Benzolpolycarbonsäure oder eines Derivates davon, und des Lösungsmittels, d. h. des nach Druchgang des 1. Reaktors zurückgeführten Produktes, jeweils in $m^3$, ist, A(Reaktor) die Querschnittsfläche des Reaktors in $m^2$, und t die Zeit in Stunden,

in der V(Zulauf) den Querschnitt A passiert, bedeuten. Die Leerrohrgeschwindigkeit ist daher ein Maß für die Strömungsgeschwindigkeit im Reaktor.

Im Rahmen des erfindungsgemäßen Verfahrens wird die Hydrierung im Allgemeinen bei einer Temperatur von 50 bis 250 °C, vorzugsweise 70 bis 180 °C durchgeführt. Die dabei verwendeten Drücke liegen in der Regel bei oberhalb von 10 bar, vorzugsweise zwischen 20 bis 80 bar, besonders bevorzugt zwischen 30 und 50 bar.

Die vorliegende Erfindung betrifft daher bevorzugt das erfindungsgemäße Verfahren, wobei die Hydrierung bei einer Temperatur von 50 bis 250 °C, vorzugsweise 70 bis 180 °C und einem Druck oberhalb von 10 bar, vorzugsweise zwischen 20 bis 80 bar, besonders bevorzugt zwischen 30 und 50 bar, durchgeführt wird.

Das erfindungsgemäße Verfahren kann entweder kontinuierlich oder diskontinuierlich durchgeführt werden, wobei die kontinuierliche Verfahrensdurchführung bevorzugt ist.

Vorzugsweise führt man das erfindungsgemäße Verfahren in Rieselreaktoren oder in gefluteter Fahrweise nach der Festbettfahrweise durch. Das Wasserstoff enthaltende Gas kann dabei sowohl im Gleichstrom mit der Lösung des zu hydrierenden Edukts als auch im Gegenstrom über den Katalysator geleitet werden. Die Hydrierung kann auch diskontinuierlich nach der Batchfahrweise durchgeführt werden.

Bei der kontinuierlichen Verfahrensführung beträgt die Menge der wenigstens einen zur Hydrierung vorgesehenen Benzolpolycarbonsäure oder einem Derivat davon vorzugsweise 0,05 bis 3 kg pro Liter Katalysator pro Stunde, weiter bevorzugt 0,1 bis 1 kg pro Liter Katalysator pro Stunde.

Als Hydriergase können beliebige Wasserstoff in freier Form enthaltende Gase verwendet werden, die keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Das erfindungsgemäße Verfahren kann in Ab- oder Anwesenheit eines Lösungs- oder Verdünnungsmittels durchgeführt werden, d. h. es ist nicht erforderlich, das Verfahren in Lösung durchzuführen.

Vorzugsweise wird jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit der wenigstens einen Benzolpolycarbonsäure oder einem Derivat davon eine homogene Lösung zu bilden.

Beispielsweise können die Lösungs- oder Verdünnungsmittel auch Wasser enthalten. Beispielsweise ist ein Lösungs- oder Verdünnungsmittel ausgewählt aus der Gruppe bestehend aus geradkettigen oder cyclischen Ethern, wie beispielsweise Tetrahydrofuran oder Dioxan, aliphatischen Alkoholen, in denen der Alkylrest vorzugsweise 1 bis 10 Kohlenstoffatome, insbesondere 3 bis 6 Kohlenstoffatome aufweist, beispielsweise i-Propanol, n-Butanol, i-Butanol, n-Hexanol und Mischungen davon.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 Gew.-%igen Lösung der wenigstens einen zur Hydrierung vorgesehenen Benzolpolycarbonsäure oder eines Derivates davon führen.

Besonders bevorzugt wird im Rahmen des erfindungsgemäßen Verfahrens das bei der Hydrierung gebildete Produkt, also die entsprechende Cyclohexanpolycarbonsäure oder ein Derivat davon als Lösungsmittel eingesetzt, gegebenenfalls neben anderen Lösungs- oder Verdünnungsmitteln. In jedem Fall kann ein Teil des im Verfahren gebildeten Produkts der noch zu hydrierenden Benzolpolycarbonsäure oder des Derivats davon beigemischt werden. Bezogen auf das Gewicht der zur Hydrierung vorgesehenen Verbindung wird vorzugsweise die 1- bis 30-fache, besonders bevorzugt die 5- bis 20-fache, insbesondere die 5- bis 10-fache Menge, des Umsetzungsproduktes als Lösungs- oder Verdünnungsmittel zugemischt.

Bei der erfindungsgemäß erhaltenen wenigstens einen Cyclohexanpolycarbonsäure oder einem Derivat davon handelt es sich bevorzugt um Verbindungen der Formel (I),

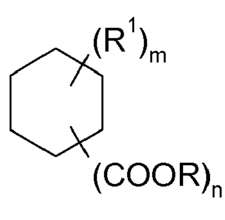

(I)

worin

$R^1$ $C_1$-$C_4$-Alkyl,
m 0, 1, 2 oder 3,
n 2, 3 oder 4, und

R Wasserstoff, $C_4$-$C_{12}$-Alkyl

bedeuten.

Um diese Verbindungen der allgemeinen Formel (I) zu erhalten, werden erfindungsgemäß als Edukte die entsprechenden aromatischen Verbindungen der allgemeinen Formel (II) eingesetzt:

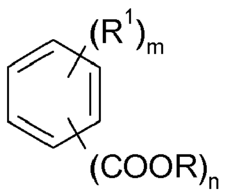

(II)

worin

$R^1$ $C_1$-$C_4$-Alkyl,
m 0, 1, 2 oder 3,
n 2, 3 oder 4, und
R Wasserstoff, $C_4$-$C_{12}$-Alkyl

bedeuten.

Die Bedeutungen von $R^1$, R, m und n in den allgemeinen Formeln (I) und (II) werden im Folgenden gemeinsam diskutiert, wobei dem Fachmann bewusst ist, dass sich die Verbindungen der Formeln (I) und (II) durch die Anzahl der Ring-Wasserstoff-Atome unterscheiden.

Wenn m 2 oder 3 ist, können die Reste $R^1$ gleich oder verschieden sein. Die $C_1$-$C_4$-Alkylgruppen können geradkettig oder verzweigt sein. Wenn $R^1$ für eine Alkylgruppe steht, handelt es sich bevorzugt um Methyl-, Ethyl-, n-Propyl-, i-Propyl, n-Butyl, i-Butyl, sek.-Butyl oder tert.-Butyl. Bevorzugt ist m 0, d. h. es liegen keine $C_1$-$C_4$-Alkyl-Substituenten vor, sondern ausschließlich Wasserstoffatome, so dass ein aromatischer Benzolring (allgemeine Formel II) bzw. ein gesättigter Cyclohexylring (allgemeine Formel (I)) vorliegt.

Die n Reste R können gleich oder verschieden sein. Für den Fall, dass R Wasserstoff bedeutet, liegt eine Cyclohexanpolycarbonsäure bzw. Benzolpolycarbonsäure vor. Die $C_4$-$C_{12}$-Alkylgruppen können geradkettig oder verzweigt sein. R ist bevorzugt ein $C_6$-$C_{12}$-Alkyl, ganz besonders bevorzugt $C_8$-$C_{10}$-Alkyl. Beispiele für derartige Alkylgruppen sind n-Butyl, i-Butyl, sek.-Butyl oder tert.-Butyl, n-Pentyl, n-Hexyl, n-Octyl, 2-Ethyl-hexyl, n-Nonyl, iso-Nonyl, n-Decyl, iso-Decyl, n-Undecyl, iso-Undecyl, n-Dodecyl, iso-Dodecyl, n-Tridecyl, iso-Tridecyl, Stearyl, und n-Eicosyl.

Werden erfindungsgemäß Anhydride der wenigstens einen Benzolpolycarbonsäure oder eines Derivates davon eingesetzt, so sind zwei Carbonsäuregruppen unter Abspaltung eines Moleküls $H_2O$ miteinander verbunden. Dabei können die beiden Carbonsäuregruppen in einem Molekül (intramolekular) oder zwei Molekülen (Intermolekular) vorliegen. Dies gilt für die Edukte gemäß der allgemeinen Formel (II) und für die Produkte gemäß der allgemeinen Formel (I) und ist dem Fachmann bekannt.

Bei den Alkylgruppen kann es sich jeweils um einzelne Isomere der genannten Alkylgruppen oder um Gemische verschiedener Alkylgruppen handeln. Die verschiedenen Alkylgruppen können verschiedene Isomere mit derselben Zahl von Kohlenstoffatomen sein und/oder Alkylgruppen, die eine verschiedene Anzahl von Kohlenstoffatomen aufweisen.

Bei den erfindungsgemäß erhaltenen Cyclohexanpolycarbonsäuren oder Derivaten davon der allgemeinen Formel (I) handelt es sich insbesondere um Mono-, Di-, Tri-, Tetraester und Anyhdride der Cyclohexanpolycarbonsäuren. Bevorzugt sind alle Carbonsäuregruppen verestert. Die eingesetzten Ester sind Alkylester, wobei bevorzugte Alkylgruppen R bereits vorstehend genannt sind.

Bevorzugt werden erfindungsgemäß Cyclohexanpolycarbonsäure-Derivate ausgewählt aus der Gruppe bestehend aus kernhydrierten Mono- und Dialkylestern der Phthalsäure, Isophthalsäure und Terephthalsäure, kernhydrierten Mono-, Di- und Trialkylestem der Trimellitsäure, der Trimesinsäure und der Hemimellitsäure oder Mono-, Di-, Tri- und Tetraalkylestern der Pyrromellitsäure erhalten, wobei die Alkylgruppen R die oben genannten Bedeutungen haben.

Die erfindungsgemäß bevorzugt eingesetzten Benzolpolycarbonsäuren sind insbesondere ausgewählt aus der Gruppe bestehend aus Phthalsäure, Isophthalsäure, Terephthalsäure, Trimellitsäure, Trimesinsäure, Hemimellitsäure, Pyromeliltsäure und Mischungen davon. Ganz besonders bevorzugt wird Phthalsäure eingesetzt. Die vorstehend genannten Säuren sind kommerziell erhältlich.

Weiter bevorzugt werden erfindungsgemäß Benzolpolycarbonsäureester der allgemeinen Formel (II) eingesetzt. Diese werden beispielsweise erhalten, indem wenigstens eine Benzolpolycarbonsäure ausgewählt aus der Gruppe

bestehend aus Phthalsäure, Isophthalsäure, Terephthalsäure, Trimellitsäure, Trimesinsäure, Hemimellitsäure, Pyromelitsäure und Mischungen davon, mit entsprechenden Alkoholen R-OH umgesetzt werden.

Als Alkohole werden bevorzugt die den Resten R der Cyclohexanpolycarbonsäure-Derivate der Formel I entsprechenden Alkohole eingesetzt.

Bevorzugt werden somit lineare oder verzweigte Alkohole mit $C_4$-$C_{12}$-Alkylresten eingesetzt. Bei den zur Veresterung mit den Benzolpolycarbonsäuren eingesetzten Alkoholen kann es sich jeweils um die den vorstehend genannten Resten R entsprechenden einzelnen Isomere der Alkohole oder um Gemische verschiedener Alkohole mit isomeren Alkylresten mit derselben Zahl von Kohlenstoffatomen handeln und/oder um Gemische verschiedener Alkohole mit unterschiedlicher Zahl der Kohlenstoffatome.

Die zur Umsetzung mit den Benzolpolycarbonsäuren geeigneten Alkohole oder Alkoholgemische können nach allen dem Fachmann bekannten Verfahren hergestellt werden. Geeignete Verfahren zur Herstellung von Alkoholen oder Verfahrensschritte, die bei der Herstellung von Alkoholen angewendet werden, sind zum Beispiel:

Hydroformylierung mit anschließender Hydrierung der gebildeten Aldehyde, zum Beispiel wie in WO 92/13818 offenbart;

Hydrierung von Aldolprodukten, zum Beispiel wie in DE-A 102 51 311 offenbart;

Hydratisierung von Alkenen, zum Beispiel wie in US 5,136,108 offenbart.

Hydrierung von Carbonsäuren und Carbonsäureestern, insbesondere Fettsäuren und Fettsäureestern, zum Beispiel wie in US 5,463,143 offenbart.

Hydrierung von ungesättigten Alkoholen oder von Carbonylverbindungen, zum Beispiel wie in EP-A 0 394 842 offenbart;

Hydrierung von Epoxiden, zum Beispiel wie in GB-A 879 803 offenbart;

Verfahren umfassend einen Telomerisationsschritt, zum Beispiel wie in US 3,091,628 offenbart;

Verfahren umfassend einen Isomerisierungsschritt, zum Beispiel wie in DE-A 42 28 887 offenbart;

Hydrolyse von Sulfaten, zum Beispiel wie in GB-A 1,165,309 offenbart;

Umsetzung von Dienen mit Aminen, zum Beispiel wie in DE-A 44 31 528 offenbart;

Enzymatische Herstellung von Alkoholen, zum Beispiel wie in WO 93/24644 offenbart;

Selektive Hydrierung von Dienen, zum Beispiel wie in US 3,203,998 offenbart;

Herstellung von Alkoholen aus Nitrilen, zum Beispiel wie in EP-A 0 271 092 offenbart;

Herstellung von Alkoholen durch Umsetzung von Alkinen, zum Beispiel wie in RU 205 9597-C1 offenbart; und

Hydrogenolyse von substituierten Tetrahydropyranen, zum Beispiel wie in GB 1,320,188 offenbart.

Dem Fachmann sind weitere Verfahren zur Herstellung von Alkoholen bekannt, die ebenfalls zur Veresterung mit Benzolpolycarbonsäuren geeigneten Alkoholen oder Alkoholgemischen eingesetzt werden können.

Bevorzugt eingesetzte Alkohole sind - wie vorstehend erwähnt - Alkohole, die $C_4$-$C_{12}$-Alkylreste aufweisen. Insbesondere die längerkettigen $C_5$-$C_{12}$-Alkohole bzw. Alkoholgemische, die diese Alkohole enthalten, werden besonders bevorzugt durch katalytische Hydroformylierung (auch als Oxoreaktion bezeichnet) von Olefinen und anschließende Hydrierung der gebildeten Aldehyde hergestellt.

Geeignete Hydroformylierungsverfahren sind dem Fachmann bekannt und sind in den vorstehend genannten Dokumenten offenbart. Die in den genannten Dokumenten offenbarten Alkohole und Alkoholgemische können mit den vorstehend genannten Benzolpolycarbonsäuren zu den gewünschten Benzolpolycarbonsäurealkylestern bzw. -estergemischen umgesetzt werden. $C_5$-Alkohole bzw. Gemische, die $C_5$-Alkohole, besonders bevorzugt n-Pentanol enthalten, können zum Beispiel durch Hydroformylierung von Butadien in Anwesenheit einer wässrigen Lösung einer Rhodiumverbindung und eines Phosphins als Katalysator hergestellt werden. Ein solches Verfahren ist zum Beispiel in EP-A 0

643 031 offenbart.

Geeignete $C_7$-Alkoholmischungen, die zur Veresterung mit den Benzolpolycarbonsäuren eingesetzt werden können, sind zum Beispiel in JP-A 2000/319 444 offenbart. Die Herstellung der $C_7$-Alkoholmischung erfolgt durch Hydroformylierung mit anschließender Hydrierung der gebildeten Aldehyde.

Mischungen enthaltend $C_8$-Alkohole und deren Herstellungsverfahren sind zum Beispiel in GB-A 721 540 offenbart, worin ein Verfahren zur Herstellung von Isooctylalkoholen ausgehend von Heptenen mittels Hydroformylierung und anschließender Hydrierung beschrieben wird. Ein beispielhaft genanntes, weiteres Dokument, das die Herstellung von $C_7$-Alkoholen bzw. diese Alkohole enthaltenden Mischungen offenbart, ist DE-A 195 30 414.

$C_9$-Alkohole bzw. Mischungen enthaltend $C_9$-Alkohole werden bevorzugt durch Dimerisierung von Butenen, Hydroformylierung der erhaltenen Octene und anschließende Hydrierung des erhaltenen $C_9$-Aldehyds hergestellt.

Geeignete Verfahren und $C_9$-Alkohle enthaltende Mischungen sind zum Beispiel in WO 92/13818 offenbart.

$C_{10}$-Alkohole und Mischungen enthaltend diese Alkohole sind zum Beispiel in WO 2003/66642 offenbart.

$C_{12}$-Alkohole bzw. Mischungen enthaltend $C_{12}$-Alkohole, insbesondere Trimethylnonanol, und ein Verfahren zu dessen Herstellung sind zum Beispiel in WO 98/03462 offenbart.

Besonders bevorzugt werden erfindungsgemäß Dialkylester der vorstehend genannten Cyclohexandicarbonsäuren, insbesondere 1,2-, 1,3- oder 1,4-Dialkylester und ganz besonders bevorzugt 1,2-Dialkylester erhalten. Dabei können Dialkylester erhalten werden bzw. die entsprechenden Benzoldicarbonsäuredialkylester eingesetzt werden, worin beide Estergruppen der Dialkylester dieselben Alkylreste tragen, sowie Estergruppen, worin die beiden Estergruppen der Dialkylester unterschiedliche Alkylgruppen tragen. Beispiele für gemischte und nicht gemischte Dialkylester sind bereits vorstehend genannt. Weiterhin ist es möglich, dass die Alkylgruppen zwar die gleiche Kohlenstoffatomanzahl aufweisen, jedoch geradkettig sind oder unterschiedliche Verzweigungen aufweisen und somit Isomerengemische bilden. Solche Isomerengemische können auch eingesetzt werden, wenn die Kohlenstoffanzahl der Alkylgruppen der Dialkylester unterschiedlich ist. Der Anteil der verschiedenen Isomeren der Alkylgruppen ergibt sich im Allgemeinen aus der Zusammensetzung der Alkohole, die zur Veresterung der Benzoldicarbonsäuren eingesetzt werden, die nach Veresterung erfindungsgemäß zu den Cyclohexandicarbonsäureestern hydriert werden. Geeignete Alkoholmischungen sind vorstehend bereits genannt. Im Sinne der vorliegenden Anmeldung sind somit unter geradkettigen oder verzweigten Alkylresten mit einer bestimmten Anzahl von Kohlenstoffatomen neben den jeweils einzelnen Isomeren Isomerengemische zu verstehen, deren Zusammensetzung sich - wie vorstehend erwähnt - aus der Zusammensetzung der zur Veresterung der Benzoldicarbonsäuren eingesetzten Alkohole ergibt.

Unter geradkettigen Alkylresten sind im Sinne der vorliegenden Anmeldung ausschließlich geradkettige Alkylreste zu verstehen, jedoch auch Mischungen von Alkylresten, die überwiegend geradkettig sind.

Handelt es sich bei den Alkylresten R der Cyclohexanpolycarbonsäureester um $C_4$-Alkylreste, so werden diese durch Umsetzung der Benzolpolycarbonsäuren der Formel (II) mit R gleich Wasserstoff mit n-Butanol, iso-Butanol, sek.-Butanol oder tert.-Butanol erhalten. Dabei können zur Herstellung von Benzolpolycarbonsäureestern, worin R ein $C_4$ ist, jeweils Gemische der genannten Butanole eingesetzt werden oder einzelne Isomere. Bevorzugt werden einzelne Isomere des Butanols eingesetzt. Die Herstellung der vorstehend genannten $C_4$-Alkohole ist dem Fachmann bekannt.

Handelt es sich bei den Alkylresten R der Cyclohexanpolycarbonsäureester um $C_5$- bis $C_{12}$-Alkylreste, werden bevorzugt $C_5$- bis $C_{12}$-Alkohole eingesetzt, die Verzweigungsgrade (ISO-Index) von im Allgemeinen 0,10 bis 4, bevorzugt 0,5 bis 3, besonders bevorzugt 0,8 bis 2 und insbesondere bei 1 bis 1,5, aufweisen, d. h. im Allgemeinen handelt es sich bei den jeweiligen Alkoholen um Gemische verschiedener Isomere.

Ganz besonders bevorzugt werden $C_9$-Alkoholgemische mit einem ISO-Index vom 1 bis 2,5, insbesondere Nonanolgemische mit einem ISO-Index von 1,25 bzw. 1,6 eingesetzt. Der ISO-Index ist eine dimensionslose Größe, die mittels Gaschromatographie bestimmt wurde.

| | |
|---|---|
| Methode: | Kapillar GC |
| Apparatur: | Kapillar Gaschromatograph mit Autosampler, Split/Splitless-Injektionssystem und Flammenionisationsdetektor (FID) |
| Chemikalien: | MSTFA (N-Methyl-N-trimethylsilyltrifluoracetamid) |
| - | entsprechende Vergleichssubstanzen zur Bestimmung der Retentionszeiten |
| Probenvorbereitung: | 3 Tropfen der Probe werden in 1 ml MSTFA und für 60 Minuten bei 80 °C gehalten |
| GC Bedingungen: | Kapillarsäule Ultra-1, Länge 50 m, Innendurchmesser 0,25 mm, Filmdicke 0,1 Mikrometer, Trägergas Helium, |
| Säulenvordruck | 200 psi constant, |
| Split | 80 ml/min, |
| Septumspülung | 3 ml/min, |
| Ofentemperatur | 120 °C, 25 min, isotherm, |

(fortgesetzt)

| | |
|---|---|
| Injektortemperatur | 250 °C, |
| Detektortemperatur | 250 °C (FID), |
| Injektionsvolumen | 0,5 Mikroliter |
| Berechnung: | Die Vorgehensweise bei der Berechnung des Iso-Index wird in der folgenden Tabelle ersichtlich |

Tabelle mit beispielhafter Berechnung des Iso-Index:

| Komponente | Name | Verzweigung | Anteil in Flächen-% | Index |
|---|---|---|---|---|
| **1** | 2-Ethyl-2-methylhexanol-1 | 2 | 1,00 | 0,0200 |
| **2** | 2-Ethyl-4-methylhexanol-1 | 2 | 1,00 | 0,0200 |
| **3** | 2-Ethyl-4-methylhexanol-1 | 2 | 1,00 | 0,0200 |
| **4** | 2-Propyl-3-methylpentanol-1 | 2 | 1,00 | 0,0200 |
| **5** | 2-Propyl-hexanol-1 | 1 | 1,00 | 0,0100 |
| **6** | 2,5-Dimethylheptanol-1 | 2 | 1,00 | 0,0200 |
| **7** | 2,3-Dimethylheptanol-1 | 2 | 1,00 | 0,0200 |
| **8** | 2,3,4-Trimethylhexanol-1 | 3 | 1,00 | 0,0300 |
| **9** | 2-Ethylheptanol-1 | 1 | 1,00 | 0,0100 |
| **10** | 3-Ethyl-4-methylhexanol-1 | 2 | 82,00 | 1,6400 |
| **11** | 3-Ethylheptanol-1 | 1 | 1,00 | 0,0100 |
| **12** | 2-Methyloctanol-1 | 1 | 1,00 | 0,0100 |
| **13** | 4,5-Dimethylheptanol-1 | 2 | 1,00 | 0,0200 |
| **14** | 4,5-Dimethylheptanol-1 | 2 | 1,00 | 0,0200 |
| **15** | 4-Methyloctanol-1 | 1 | 1,00 | 0,0100 |
| **15a** | 7-Methyloctanol-1 | 1 | 1,00 | 0,0000 |
| **16** | 6-Methyloctanol-1 | 1 | 1,00 | 0,0100 |
| **17** | Nonanol-1 | 0 | 1,00 | 0,0000 |
| | Unbekannte Komponente | 2 | 1,00 | 0,0200 |
| | Summe | | 99,00 | 1,9000 |
| | | | Iso-Index: | 1,9200 |

Die $C_5$- bis $C_{12}$-Alkohole werden gemäß den vorstehend genannten Verfahren hergestellt. Zur Herstellung von Benzolpolycarbonsäureestern, worin R ein $C_9$-Alkyrest ist, wird besonders bevorzugt ein Nonanol-Gemisch eingesetzt, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind. Unter sonstigen Komponenten sind im Allgemeinen Nonanole mit mehr als drei Verzweigungen, Decanole oder Octanole zu verstehen, wobei die Summe der genannten Komponenten 100 Gew.-% ergibt.

Die vorliegende Erfindung betrifft daher bevorzugt das erfindungsgemäße Verfahren, wobei es sich bei dem wenigstens einen Derivat der Benzolpolycarbonsäure um Mono-, Di-, Tri-, Tetraester und Anyhdride der Benzolpolycarbonsäure handelt. Bevorzugt sind alle Carbonsäuregruppen verestert. Die eingesetzten Ester sind Alkylester, wobei bevorzugte Alkylgruppen R bereits vorstehend genannt sind.

Weiter bevorzugt betrifft die vorliegende Erfindung das erfindungsgemäße Verfahren, wobei es sich bei dem

wenigstens einen Derivat einer Benzolpolycarbonsäure um Mono-, Di-, Tri-, Tetraester der Benzolpolycarbonsäure, wobei diese durch Umsetzung mit einem Nonanol-Gemisch, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind, wobei die Summe der genannten Komponenten 100 Gew.-% ergibt, umgesetzt worden ist, handelt.

Eine besonders bevorzugte Ausführungsform eines Nonanol-Gemisches, das zur Herstellung von bevorzugt verwendeten Benzolpolycarbonsäurederivaten eingesetzt wird, weist die folgende Zusammensetzung auf:

1.73 bis 3,73 Gew.-%, bevorzugt 1,93 bis 3,53 Gew.-%, besonders bevorzugt 2,23 bis 3,23 Gew.-% 3-Ethyl-6-methyl-hexanol;
0,38 bis 1,38 Gew.-%, bevorzugt 0,48 bis 1,28 Gew.-%, besonders bevorzugt 0,58 bis 1,18 Gew.-% 2,6 Dimethylheptanol;
2,78 bis 4,78 Gew.-%, bevorzugt 2,98 bis 4,58 Gew.-%, besonders bevorzugt 3,28 bis 4,28 Gew.-% 3,5-Dimethylheptanol;
6,30 bis 16,30 Gew.-%, bevorzugt 7,30 bis 15,30 Gew.-%, besonders bevorzugt 8,30 bis 14,30 Gew.-% 3,6-Dimethylheptanol;
5.74 bis 11,74 Gew.-%, bevorzugt 6,24 bis 11,24 Gew.-%, besonders bevorzugt 6,74 bis 10,74 Gew.-% 4,6-Dimethylheptanol;
1,64 bis 3,64 Gew.-%, bevorzugt 1,84 bis 3,44 Gew.-%, besonders bevorzugt 2,14 bis 3,14 Gew.-% 3,4,5-Trimethylhexanol;
1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;
4,00 bis 10,00 Gew.-%, bevorzugt 4,50 bis 9,50 Gew.-%, besonders bevorzugt 5,00 bis 9,00 Gew.-% 3,4-Dimethylheptanol;
0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
1,21 bis 5,21 Gew.-%, bevorzugt 1,71 bis 4,71 Gew.-%, besonders bevorzugt 2,21 bis 4,21 Gew.-% 4,5-Dimethylheptanol;
1,55 bis 5,55 Gew.-%, bevorzugt 2,05 bis 5,05 Gew.-%, besonders bevorzugt 2,55 bis 4,55 Gew.-% 5,6-Dimethylheptanol;
1,63 bis 3,63 Gew.-%, bevorzugt 1,83 bis 3,43 Gew.-%, besonders bevorzugt 2,13 bis 3,13 Gew.-% 4-Methyloctanol;
0,98 bis 2,98 Gew.-%, bevorzugt 1,18 bis 2,78 Gew.-%, besonders bevorzugt 1,48 bis 2,48 Gew.-% 5-Methyloctaonol;
0,70 bis 2,70 Gew.-%, bevorzugt 0,90 bis 2,50 Gew.-%, besonders bevorzugt 1,20 bis 2,20 Gew.-% 3,6,6-Trimethylhexanol;
1,96 bis 3,96 Gew.-%, bevorzugt 2,16 bis 3,76 Gew.-%, besonders bevorzugt 2,46 bis 3,46 Gew.-% 7-Methyloctanol;
1,24 bis 3,24 Gew.-%, bevorzugt 1,44 bis 3,04 Gew.-%, besonders bevorzugt 1,74 bis 2,74 Gew.-% 6-Methyloctanol;
0,1 bis 3 Gew.-%, bevorzugt 0,2 bis 2 Gew.-%, besonders bevorzugt 0,3 bis 1 Gew.-% n-Nonanol;
25 bis 35 Gew.-%, bevorzugt 28 bis 33 Gew.-%, besonders bevorzugt 29 bis 32 Gew.-% sonstige Alkohole mit 9 und 10 Kohlenstoffatomen;

wobei die Gesamtsumme der genannten Komponenten 100 Gew.-% ergibt.

Ein solches Isononanol-Gemisch liegt verestert mit Phthalsäure im Diisononylphthalat der CAS Nr. 68515-48-0 vor, aus dem der Cyclohexan-1,2-dicarbonsäurediisononylester mit entsprechender Isononyl-Komponente durch das erfindungsgemäße Verfahren durch Hydrierung des aromatischen Kerns erzeugt werden kann. Solche Isononanol-Gemische können über den Weg der Zeolith-katalysierten Oligomerisierung von $C_2$-, $C_3$- und $C_4$-Olefingemischen, dem sogenannten Polygas-Prozess, Gewinnung einer $C_8$-Fraktion aus dem Oligomerisat und deren anschließende Hydroformylierung und Hydrierung erhalten werden.

Eine weitere besonders bevorzugte Ausführungsform eines Nonanol-Gemisches, das zur Herstellung von bevorzugt verwendeten Benzolpolycarbonsaure-Derivaten eingesetzt wird, weist die folgende Zusammensetzung auf:

6,0 bis 16,0 Gew.-%, bevorzugt 7,0 bis 15,0 Gew.-%, besonders bevorzugt 8,0 bis 14,0 Gew.-% n-Nonanol;
12,8 bis 28,8 Gew.-%, bevorzugt 14,8 bis 26,8 Gew.-%, besonders bevorzugt 15,8 bis 25,8 Gew.-% 6-Methyloctanol;

12,5 bis 28,8 Gew.-%, bevorzugt 14,5 bis 26,5 Gew.-%, besonders bevorzugt 15,5 bis 25,5 Gew.-% 4-Methyloctanol;
3,3 bis 7,3 Gew.-%, bevorzugt 3,8 bis 6,8 Gew.-%, besonders bevorzugt 4,3 bis 6,3 Gew.-% 2-Methyloctanol;
5,7 bis 11,7 Gew.-%, bevorzugt 6,3 bis 11,3 Gew.-%, besonders bevorzugt 6,7 bis 10,7 Gew.-% 3-Ethylheptanol;
1,9 bis 3,9 Gew.-%, bevorzugt 2,1 bis 3,7 Gew.-%, besonders bevorzugt 2,4 bis 3,4 Gew.-% 2-Ethylheptanol;
1.7 bis 3,7 Gew.-%, bevorzugt 1,9 bis 3,5 Gew.-%, besonders bevorzugt 2,2 bis 3,2 Gew.-% 2-Propylhexanol;
3,2 bis 9,2 Gew.-%, bevorzugt 3,7 bis 8,7 Gew.-%, besonders bevorzugt 4,2 bis 8,2 Gew.-% 3,5-Dimethylheptanol;
6,0 bis 16,0 Gew.-%, bevorzugt 7,0 bis 15,0 Gew.-%, besonders bevorzugt 8,0 bis 14,0 Gew.-% 2,5-Dimethylheptanol;
1.8 bis 3,8 Gew.-%, bevorzugt 2,0 bis 3,6 Gew.-%, besonders bevorzugt 2,3 bis 3,3 Gew.-% 2,3-Dimethylheptanol;
0,6 bis 2,6 Gew.-%, bevorzugt 0,8 bis 2,4 Gew.-%, besonders bevorzugt 1,1 bis 2,1 Gew.-% 3-Ethyl-4-methylhexanol;
2,0 bis 4,0 Gew.-%, bevorzugt 2,2 bis 3,8 Gew.-%, besonders bevorzugt 2,5 bis 3,5 Gew.-% 2-Ethyl-4-methylhexanol;
0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;

wobei die Gesamtsumme der genannten Komponenten 100 Gew.-% ergibt.

Ein solches Isononanol-Gemisch liegt verestert mit Phthalsäure im Diisononylphthalat der CAS Nr. 28553-12-0 vor, aus dem der Cyclohexan-1,2-dicarbonsäurediisononylester mit entsprechender Isononyl-Komponente durch die erfindungsgemäße Hydrierung des aromatischen Kerns erzeugt werden kann. Solche Isononanol-Gemische können über den Weg der Dimerisierung von vorwiegend n-Butenen zu Octen-Gemischen mittels Nickel-haltigen Katalysatoren, beispielsweise nach dem Verfahren der WO 95/14647, anschließende Hydroformylierung des erhaltenen Octen-Gemisches, vorzugsweise Kobalt-katalysierte Hydroformylierung, und Hydrierung erhalten werden.

Ganz besonders bevorzugte Produkte des erfindungsgemäßen Verfahrens sind ausgewählt aus der Gruppe bestehend aus Cyclohexan-1,2-dicarbonsäuredi-n-octylester, Cyclohexan-1,2-dicarbonsäurediisooctylester, Cyclohexan-1,2-dicarbonsäuredi- (2-ethylhexyl)-ester, Cyclohexan-1,2-dicarbonsäuredi-n-nonylester, Cyclohexan-1,2-dicarbonsäurediisononylester, Cyclohexan-1,2-dicarbonsäuredi-(2-propylheptyl)-ester, Cyclohexan-1,2-dicarbonsäuredi-n-decyl-ester, Cyclohexan-1,2-dicarbonsäurediisodecylester und Mischungen davon.

Weitere erfindungsgemäß bevorzugt erhaltene Produkte sind die in der WO 99/32427 offenbarten, im Folgenden nochmals aufgelisteten Cyclohexan-1,2-dicarbonsäureester:

Cyclohexan-1,2-dicarbonsäuredi(isononyl)ester, erhältlich durch die erfindungsgemäße Hydrierung eines Di(isononyl)phthalats mit der CAS Nr. 68515-48-0;

Cyclohexan-1,2-dicarbonsäuredi(isononyl)ester, erhältlich durch die erfindungsgemäße Hydrierung eines Di(isononyl)phthalats mit der CAS Nr. 28553-12-0, basierend auf n-Buten;

Cyclohexan-1,2-dicarbonsäuredi(isononyl)ester, erhältlich durch die erfindungsgemäße Hydrierung eines Di(isononyl)phthalats mit der CAS Nr. 28553-12-0 basierend auf Isobuten;

ein 1,2-Di-$C_9$-Ester der Cyclohexandicarbonsäure, erhältlich durch die erfindungsgemäße Hydrierung eines Di(nonyl)phthalats mit der CAS Nr. 68515-46-8;

ein Cyclohexan-1,2-dicarbonsäuredi(isodecyl)ester erhältlich durch die erfindungsgemäße Hydrierung eines Di(isodecyl)phthalats mit der CAS Nr. 68515-49-1;

ein 1,2-Di(isodecyl)cyclohexandicarbonsäureester, erhältlich durch die erfindungsgemäße Hydrierung eines Di(isodecyl)phthalats, das hauptsächlich aus Di-(2-propylheptyl)phthalat besteht;

Des Weiteren sind auch die kommerziell erhältlichen Benzolcarbonsäureester mit den Handelsnamen Jayflex DINP (CAS Nr. 68515-25 48-0), Jayflex DIDP (CAS Nr. 68515-49-1), Palatinol 9-P, Vestinol 9 (CAS Nr. 28553-12-0), Palatinol N (CAS Nr. 28553-12-0), Jayflex DIOP (CAS Nr. 27554-26-3), Palatinol AH (CAS Nr. 117-81-7) und Palatinol Z (CAS Nr. 26761-40-0) geeignete Edukte für das erfindungsgemäße Verfahren.

Die erfindungsgemäß hergestellten Cyclohexanpolycarbonsäuren oder Derivate davon zeichnen sich gegenüber kommerziell verfügbaren Cyclohexanpolycarbonsäuren oder Derivaten davon durch einen geringeren Anteil an Nebenkomponenten aus, insbesondere an der Nebenkomponente Hexahydrophthalid und Isononylalkohol, und weisen dadurch bei der Verwendung als Weichmacher vorteilhaftere anwendungstechnische Eigenschaften, wie eine geringere Flüchtigkeit und eine bessere Verträglichkeit mit Kunststoffen, beispielsweise PVC, auf.

Die vorliegende Erfindung betrifft daher des Weiteren Derivate von Cyclohexanpolycarbonsäuren ausgewählt aus der Gruppe bestehend aus Cyclohexan-1,2-dicarbonsäuredi-n-octylester, Cyclohexan-1,2-dicarbonsäurediisooctylester, Cyclohexan-1,2-dicarbonsäuredi- (2-ethylhexyl)-ester, Cyclohexan-1,2-dicarbonsäuredi-n-nonylester, Cyclohexan-1,2-dicarbonsäurediisononylester, Cyclohexan-1,2-dicarbonsäuredi-(2-propylheptyl)-ester, Cyclohexan-1,2-dicarbonsäuredi-n-decyl-ester, Cyclohexan-1,2-dicarbonsäurediisodecylester, jeweils erhalten durch die erfindungsgemäße Hydrierung, Cyclohexan-1,2-dicarbonsäuredi(isononyl)ester, erhältlich durch die erfindungsgemäße Hydrierung eines Di(isononyl)phthalats mit der CAS Nr. 68515-48-0, Cyclohexan-1,2-dicarbonsäuredi(isononyl)ester, erhältlich durch die erfindungsgemäße Hydrierung eines Di(isononyl)phthalats mit der CAS Nr. 28553-12-0, basierend auf n-Buten, Cyclohexan-1,2-dicarbonsäuredi(isononyl)ester, erhältlich durch die erfindungsgemäße Hydrierung eines Di(isononyl)phthalats mit der CAS Nr. 28553-12-0 basierend auf Isobuten, ein 1,2-Di-$C_9$-Ester der Cyclohexandicarbonsäure, erhältlich durch die erfindungsgemäße Hydrierung eines Di(nonyl)phthalats mit der CAS Nr. 68515-46-8, ein Cyclohexan-1,2-dicarbonsäuredi(isodecyl)ester erhältlich durch die erfindungsgemäße Hydrierung eines Di(isodecyl)phthalats mit der CAS Nr. 68515-49-1, ein 1,2-Di(isodecyl)cyclohexandicarbonsäureester, erhältlich durch die erfindungsgemäße Hydrierung eines Di(isodecyl)phthalats, das hauptsächlich aus Di-(2-propylheptyl)phthalat besteht und Mischungen davon.

Die erfindungsgemäß hergestellte wenigstens eine Cyclohexanpolycarbonsäure oder ein Derivat davon weist in einer bevorzugten Ausführungsform einen Gehalt an Hexahydrophthalid von höchstens 0,06 Fl.-% bevorzugt weniger als 0,04 Fl.-%, besonders bevorzugt 0,01 bis 0,03 Fl.-%, auf.

Die erfindungsgemäß hergestellte wenigstens eine Cyclohexanpolycarbonsäure oder ein Derivat davon weist in einer bevorzugten Ausführungsform einen Gehalt an Isonanol von höchstens 0,2 Fl.-%, bevorzugt weniger als 0,15 Fl.-%, besonders bevorzugt 0,05 Fl.-% bis 0,13 Fl.-%, auf, falls erfindungsgemäß ein Cyclohexanpolycarbonsäureisononanolester hergestellt wird.

Dadurch sind die erfindungsgemäß hergestellten Cyclohexanpolycarbonsäuren oder Derivate davon besonders gut für Anwendungen in Kontakt mit dem Menschen geeignet, z. B. für Kinderspielzeug, Lebensmittelverpackungen oder medizinische Artikel.

Die vorliegende Erfindung betrifft daher des Weiteren die Verwendung der erfindungsgemäß hergestellten Cyclohexanpolycarbonsäuren oder Derivate davon in Anwendungen in Kontakt mit dem Menschen, insbesondere in Kinderspielzeug, Lebensmittelverpackungen oder in medizinischen Artikeln.

Beispiele

Im Folgenden soll nunmehr das erfindungsgemäße Verfahren anhand einiger Ausführungsbeispiele näher erläutert werden. Die Versuche 4-6 wurden erfindungsgemäß durchgeführt, bei den Versuchen 1-3 handelt es sich um Vergleichsversuche, in allen Fällen ist eine Verringerung der unerwünschten Nebenprodukte (Isononanol und Hexahydrophthalid) um etwa 50% oder mehr zu beobachten. Entsprechend liegt der Gehalt des Wertproduktes (Diisononylcyclohexandicarboxylat) in den erfindungsgemäßen Versuchen 4-6 bei >99,5%.

Katalysator 1 wird gemäß WO2011/082991, Beispiel 1, hergestellt.

Katalysator 2 wird gemäß DE19624485, Herstellungsbeispiel, hergestellt.

Hydrierbeispiele

Die Hydrierungen werden in einer Kaskade von 8 Rohren (Innendurchmesser 6 mm, Länge 150 cm) durchgeführt. Dabei wurden die ersten sechs Rohre in Reihe als Hauptreaktor mit Umlauf betrieben, d.h. der Austrag des sechsten Rohrs wurde teilweise auf das erste Rohr zurückgeführt. Die letzten 2 Rohre wurden als Nachreaktor im geraden Durchgang betrieben. Jedes Rohr wurde mit 30 mL Katalysator befüllt.

Die Hydrierung wurde mit reinem Wasserstoff durchgeführt. Der Zulauf wurde so gewählt, dass die Katalysatorbelastung im Hauptreaktor (kg(Diisonylphtalat)/(L(Katalysator)·h) den in der nachfolgenden Tabelle angegeben Wert erreicht. Die Rückführrate wurde so gewählt, dass die Leerrohr-Geschwindigkeit im Hauptreaktor bei den in der Tabelle 2 angegebenen Werten liegt. Der Wasserstoff wurde druckgeregelt bei dem in der Tabelle 2 angegebenen Druck zugeführt. Die Reaktionstemperaturen sind ebenfalls in der Tabelle 2 angegeben.

Tabelle 2: Hydrierversuche

| Nr. | Kat. | Katalysator-belastung [kg/(L·h)] | Leerrohr-Geschwindigkeit [m/h] | T im Hauptreaktor [°C] | T im Nachreaktor [°C] | Druck [bar] |
|---|---|---|---|---|---|---|
| 1 | 2 | 0,5 | 55 | 111 | 125 | 240 |
| 2 | 1 | 0,73 | 55 | 126 | 155 | 240 |
| 3 | 1 | 0,5 | 55 | 111 | 125 | 240 |

(fortgesetzt)

| Nr. | Kat. | Katalysator-belastung [kg/(L·h)] | Leerrohr-Geschwindigkeit [m/h] | T im Hauptreaktor [°C] | T im Nachreaktor [°C] | Druck [bar] |
|---|---|---|---|---|---|---|
| 4 | 1 | 0,5 | 34 | 111 | 125 | 36 |
| 5 | 1 | 0,5 | 34 | 120 | 140 | 36 |
| 6 | 1 | 0,25 | 34 | 111 | 125 | 36 |
| Katalysatorbelastung: kg (Diisononylphthalat)/(L(Katalysator) Stunde) Leerrohr-Geschwindigkeit: $m^3$(Diisononylphthalat)/($m^2$(Reaktor-Querschnitt) Zeit (h)) | | | | | | |

Tabelle 3: Ergebnisse der Hydrierversuche
GC-Methode:

Säule: DB-1 30m, ID 0,32 mm, FD 0,25 µm
Temperaturprogramm: 50°C, 4 min - 5°C/min - 290°C - 28 min
Injektionsvolumen: 1 µL
Inlet-Temperatur: 300°C, Detektortemperatur (FID): 300°C
Isononanol: 13-18 min
Hexahydrophthalid: 20 min

Cyclohexan-1,2-dicarbonsäurediisononylester (DINCH): 47-54 min

| Nr. | Isononanol [GC-Flächen-%] | Hexahydrophthalid [GC-Flächen-%] | DINCH-Gehalt [GC-Flächen-%] | Summe Sonstige [GC-Flächen-%] |
|---|---|---|---|---|
| 1 | 0,073 | 0,449 | 99,2 | 0,278 |
| 2 | 0,776 | 0,181 | 98,6 | 0,443 |
| 3 | 0,077 | 0,282 | 99,4 | 0,241 |
| 4 | 0,019 | 0,097 | 99,7 | 0,184 |
| 5 | 0,028 | 0,109 | 99,6 | 0,263 |
| 6 | 0,029 | 0,113 | 99,7 | 0,158 |

## Patentansprüche

1. Verfahren zur Herstellung von wenigstens einer Cyclohexanpolycarbonsäure oder einem Derivat davon durch Inkontaktbringen wenigstens einer entsprechenden Benzolpolycarbonsäure oder eines Derivates davon mit einem Wasserstoff enthaltenden Gas in Gegenwart wenigstens eines Hydrierkatalysators, **dadurch gekennzeichnet, dass** das Inkontaktbringen bei einer Leerrohrgeschwindigkeit von höchstens 50 m/h erfolgt.

2. Verfahren nach Anspruch 1, **dadurch gekennzeichnet, dass** als Katalysator ein Schalenkatalysator, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2/g$ beträgt, und mindestens 90% der vorhandenen Poren einen Durchmesser von 6 bis 12 nm aufweisen, eingesetzt wird.

3. Verfahren nach Anspruch 1 oder 2, **dadurch gekennzeichnet, dass** das Verfahren bei einer Leerohrgeschwindigkeit von höchstens 45 m/h, ganz besonders bevorzugt höchstens 40 m/h, durchgeführt wird.

4. Verfahren nach einem der Ansprüche 1 bis 3, **dadurch gekennzeichnet, dass** das die Hydrierung bei einer Temperatur von 50 bis 250 °C, vorzugsweise 70 bis 180 °C und einem Druck oberhalb von 10 bar, vorzugsweise zwischen 20 bis 80 bar, besonders bevorzugt zwischen 30 und 50 bar, durchgeführt wird.

5. Verfahren nach einem der Ansprüche 1 bis 4, **dadurch gekennzeichnet, dass** es sich bei dem wenigstens einen Derivat der Benzolpolycarbonsäure um Mono-, Di-, Tri-, Tetraester und Anyhdride der Benzolpolycarbonsäure handelt.

6. Verfahren nach einem der Ansprüche 1 bis 5, **dadurch gekennzeichnet, dass** es sich bei dem wenigstens einen Derivat einer Benzolpolycarbonsäure um Mono-, Di-, Tri-, Tetraester der Benzolpolycarbonsäure handelt, wobei diese durch Umsetzung mit einem Nonanol-Gemisch, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind, wobei die Summe der genannten Komponenten 100 Gew.-% ergibt, erhalten werden.

7. Derivate von Cyclohexanpolycarbonsäuren ausgewählt aus der Gruppe bestehend aus Cyclohexan-1,2-dicarbonsäuredi-n-octylester, Cyclohexan-1,2-dicarbonsäurediisooctylester, Cyclohexan-1,2-dicarbonsäuredi- (2-ethylhexyl)-ester, Cyclohexan-1,2-dicarbonsäuredi-n-nonylester, Cyclohexan-1,2-dicarbonsäurediisononylester, Cyclohexan-1,2-dicarbonsäuredi-(2-propylheptyl)-ester, Cyclohexan-1,2-dicarbonsäuredi-n-decyl-ester, Cyclohexan-1,2-dicarbonsäurediisodecylester, jeweils erhalten durch das Verfahren nach einem der Ansprüche 1 bis 5, Cyclohexan-1,2-dicarbonsäuredi(isononyl)ester, erhältlich durch die Hydrierung eines Di(isononyl)phthalats mit der CAS Nr. 68515-48-0 nach einem der Ansprüche 1 bis 5" Cyclohexan-1,2-dicarbonsäuredi(isononyl)ester, erhältlich durch die Hydrierung eines Di(isononyl)phthalats mit der CAS Nr. 28553-12-0, basierend auf n-Buten, nach einem der Ansprüche 1 bis 5, Cyclohexan-1,2-dicarbonsäuredi(isononyl)ester, erhältlich durch die Hydrierung eines Di(isononyl)phthalats mit der CAS Nr. 28553-12-0 basierend auf Isobuten, nach einem der Ansprüche 1 bis 5, ein 1,2-Di-C9-Ester der Cyclohexandicarbonsäure, erhältlich durch die Hydrierung eines Di(nonyl)phthalats mit der CAS Nr. 68515-46-8 nach einem der Ansprüche 1 bis 5, ein Cyclohexan-1,2-dicarbonsäuredi(isodecyl)ester erhältlich durch die Hydrierung eines Di(isodecyl)phthalats mit der CAS Nr. 68515-49-1 nach einem der Ansprüche 1 bis 5, ein 1,2-Di(isodecyl)cyclohexandicarbonsäureester, erhältlich durch die Hydrierung eines Di(isodecyl)phthalats, das hauptsächlich aus Di-(2-propylheptyl)phthalat besteht, nach einem der Ansprüche 1 bis 5, und Mischungen davon.

8. Verwendung der Cyclohexanpolycarbonsäuren oder Derivate davon gemäß Anspruch 7 in Anwendungen in Kontakt mit dem Menschen, insbesondere in Kinderspielzeug, Lebensmittelverpackungen oder in medizinischen Artikeln.

Europäisches Patentamt
European Patent Office
Office européen des brevets

# EUROPÄISCHER RECHERCHENBERICHT

Nummer der Anmeldung
EP 12 18 7399

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2008/188601 A1 (GRASS MICHAEL [DE] ET AL) 7. August 2008 (2008-08-07)<br>* Absätze [0055], [0072] *<br>* Beispiel 2 *<br>* Anspruch 7 *<br>----- | 1-8 | INV.<br>C07C51/36<br>C07C61/08<br>C07C67/303<br>C07C69/75 |
| X | US 2007/255070 A1 (LIU ZHUFANG [US]) 1. November 2007 (2007-11-01)<br>* Ansprüche 1, 10-12 *<br>----- | 1-8 | |
| X | WO 94/29261 A1 (EASTMAN CHEM CO [US]) 22. Dezember 1994 (1994-12-22)<br>* Seite 5, Zeilen 21-34 *<br>* Beispiel 1 *<br>* Anspruch 1 *<br>----- | 1-8 | |
| X,D | WO 2011/082991 A2 (BASF SE [DE]; KOENIGSMANN LUCIA [DE]; MIRK DANIELA [DE]; HEIDEMANN THO) 14. Juli 2011 (2011-07-14)<br>* Beispiele 2.3-2.6 *<br>----- | 1-8 | |
| X | EP 1 323 700 A1 (MITSUBISHI GAS CHEMICAL CO [JP]) 2. Juli 2003 (2003-07-02)<br>* Anspruch 2 *<br>* Beispiele 30-31 *<br>----- | 1-8 | RECHERCHIERTE SACHGEBIETE (IPC)<br>C07C |
| X | EP 2 316 811 A1 (MITSUBISHI GAS CHEMICAL CO [JP]) 4. Mai 2011 (2011-05-04)<br>* Anspruch 1 *<br>* Beispiel 1 *<br>----- | 1-6 | |

Der vorliegende Recherchenbericht wurde für alle Patentansprüche erstellt

1

| Recherchenort | Abschlußdatum der Recherche | Prüfer |
|---|---|---|
| München | 1. März 2013 | Panday, Narendra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 12 18 7399

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-03-2013

| Im Recherchenbericht angeführtes Patentdokument | Datum der Veröffentlichung | Mitglied(er) der Patentfamilie | Datum der Veröffentlichung |
|---|---|---|---|
| US 2008188601 A1 | 07-08-2008 | CA 2606551 A1 | 28-12-2006 |
| | | CN 101203477 A | 18-06-2008 |
| | | DE 102005028752 A1 | 04-01-2007 |
| | | EP 1893558 A1 | 05-03-2008 |
| | | US 2008188601 A1 | 07-08-2008 |
| | | WO 2006136471 A1 | 28-12-2006 |
| US 2007255070 A1 | 01-11-2007 | KEINE | |
| WO 9429261 A1 | 22-12-1994 | CA 2165205 A1 | 22-12-1994 |
| | | CN 1099744 A | 08-03-1995 |
| | | DE 69409519 D1 | 14-05-1998 |
| | | DE 69409519 T2 | 06-08-1998 |
| | | EP 0703896 A1 | 03-04-1996 |
| | | ES 2115241 T3 | 16-06-1998 |
| | | JP 3510890 B2 | 29-03-2004 |
| | | JP H08511776 A | 10-12-1996 |
| | | SG 47884 A1 | 17-04-1998 |
| | | US 5399742 A | 21-03-1995 |
| | | WO 9429261 A1 | 22-12-1994 |
| WO 2011082991 A2 | 14-07-2011 | CN 102753266 A | 24-10-2012 |
| | | EP 2512658 A2 | 24-10-2012 |
| | | KR 20120092197 A | 20-08-2012 |
| | | US 2012296111 A1 | 22-11-2012 |
| | | WO 2011082991 A2 | 14-07-2011 |
| EP 1323700 A1 | 02-07-2003 | CN 1428324 A | 09-07-2003 |
| | | DE 60221955 T2 | 06-12-2007 |
| | | EP 1323700 A1 | 02-07-2003 |
| | | JP 4217877 B2 | 04-02-2009 |
| | | JP 2003286222 A | 10-10-2003 |
| | | JP 2009057385 A | 19-03-2009 |
| | | KR 20030022754 A | 17-03-2003 |
| | | KR 20090024762 A | 09-03-2009 |
| | | KR 20090101144 A | 24-09-2009 |
| | | TW I259177 B | 01-08-2006 |
| | | US 2003149297 A1 | 07-08-2003 |
| EP 2316811 A1 | 04-05-2011 | CN 102105428 A | 22-06-2011 |
| | | EP 2316811 A1 | 04-05-2011 |
| | | KR 20110036053 A | 06-04-2011 |
| | | TW 201008907 A | 01-03-2010 |
| | | US 2011196171 A1 | 11-08-2011 |
| | | WO 2010010869 A1 | 28-01-2010 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5286898 A
- US 5319129 A
- DE 2823165 A
- US 3027398 A
- EP 0603825 A
- EP 1042273 A
- DE 1263296 A
- DE 2519624484 A
- DE 19624485 A
- DE 19604791 A
- WO 201108299 A
- WO 2011082991 A
- WO 2004046078 A
- US 7893295 B
- DE 10225565
- DE 10146847
- WO 04009526 A
- WO 9213818 A
- DE 10251311 A
- US 5136108 A
- US 5463143 A
- EP 0394842 A
- GB 879803 A
- US 3091628 A
- DE 4228887 A
- GB 1165309 A
- DE 4431528 A
- WO 9324644 A
- US 3203998 A
- EP 0271092 A
- RU 2059597 C1
- GB 1320188 A
- EP 0643031 A
- JP 2000319444 A
- GB 721540 A
- DE 19530414 A
- WO 200366642 A
- WO 9803462 A
- WO 9514647 A
- WO 9932427 A
- DE 19624485

### In der Beschreibung aufgeführte Nicht-Patentliteratur

- Hydrogenation and Dehydrogenation. Ullmann's Encyclopedia of Industrial Chemistry. vol. 18, 483-541
- Heterogeneous Catalysis and Solid Catalysts, 2. Development and Types of Solid Catalysts,. Ullmann's Encyclopedia of Industrial Chemistry. vol. 17, 483-541
- **J. LEMAITRE et al.** Characterization of Heterogeneous Catalysts. Marcel Dekker, 1984, 310-324
- **J.W. NIEMANTSVERDRIET.** Spectroscopy in Catalysis. VCH, 1995
- **S. AMELINCKX.** Handbook of Microscopy